# EUROPÄISCHE PATENTSCHRIFT

(11) **EP 0 813 886 B1**
(45) Veröffentlichungstag und Bekanntmachung des Hinweises auf die Patenterteilung: **22.09.2004**
(21) Anmeldenummer: 97250187.8
(22) Anmeldetag: 16.06.1997
(51) Int. Cl.: A61N 1/05

(54) **Defibrillationselektrodenanordnung**
Defibrillation electrode device
Système d'électrodes de défibrillation

(30) Priorität: 18.06.1996 DE 19626352
(43) Veröffentlichungstag der Anmeldung: 29.12.1997
(73) Patentinhaber: BIOTRONIK Mess- und Therapiegeräte GmbH & Co Ingenieurbüro Berlin, 12359 Berlin (DE)
(72) Erfinder: Thong, Tran, Lake Oswego, Oregon 97035 (US); Schaldach, Max, Prof. Dr.-Ing., 91054 Erlangen (DE)
(74) Vertreter: Eisenführ, Speiser & Partner

(56) Entgegenhaltungen:
- WO-A-92/09329
- WO-A-95/08365
- WO-A-95/08366
- US-A- 4 727 877
- US-A- 5 235 978
- US-A- 5 374 279

## Beschreibung

Die Erfindung betrifft eine Defibrillationselektrodenanordnung der im Oberbegriff des Anspruchs 1 angegebenen Art.

Implantierbare automatische Defibrillatoren finden zunehmend Einsatz bei Patienten mit medikamentös nicht oder nicht zuverlässig therapierbaren Herzrhythmusstörungen, die in das plötzliche Auftreten von Herzkammerflimmern münden können. Solche Patienten bedürfen in vielen Fällen zusätzlich einer Schrittmacherunterstützung ihrer Herztätigkeit, so daß speziell auch Kombinationsgeräte mit einem Herzschrittmacher einige Verbreitung erlangt haben.

Wegen des geringeren Operationsaufwandes und -risikos setzen sich als Elektrodenanordnungen gegenüber den früheren subkutanen Flächenelektroden immer deutlicher solche Anordnungen durch, bei denen zumindest ein Teil der Elektroden auf einem transvenös endokardial zu verlegenden Katheter- bzw. Elektrodenleitungskörper angeordnet ist. Diese Elektrodenanordnungen haben in Verbindung mit Schrittmacher/Defibrillator-Kombinationsgeräten zudem den Vorteil, daß ein Teil der Elektroden sowohl als Schrittmacher- wie auch als Defibrillationselektroden genutzt werden kann oder jedenfalls Abfühl-, Schrittmacher-und Defibrillationselektroden auf ein und derselben Elektrodenleitung ausgeführt werden können, so daß das Verlegen mehrerer separater Katheter weitgehend unnötig wird.

Eine frühe Elektrodenanordnung, bei der auf subkutane Elektroden gänzlich verzichtet wird und die eine wandständig im ventrikulären Apex sowie eine in der superior vena cava anzuordnende Elektrode umfaßt, ist in der US-Patentschrift Nr. 3 942 536 beschrieben.

In der US-Patentschrift Nr. 4 727 877 ist - unter anderem-eine zur niederenergetischen Defibrillation mittels Stimulationsimpulspaaren bestimmte endokardiale Elektrodenanordnung beschrieben, die zwei Elektrodenpaare aus insgesamt drei langgestreckt zylinderförmige Elektroden umfaßt, von denen eine im Apex des rechten Ventrikels, eine zweite im oder am sinus coronaris und die dritte in der vena cava superior angeordnet ist. Die Anordnung ist auf zwei separaten Elektrodenleitungen realisiert.

Bei einer in WO 92/09329 beschriebenen, in den Ventrikel einzuschraubenden Anordnung sind neben einer Spitzenelektrode drei langgestreckte Elektrodenabschnitte auf einer einzelnen Leitung vorgesehen, die zusammen mit einer Sinus-Elektrode oder einer subkutanen Plattenelektrode eingesetzt wird. Das Vorsehen mehrerer Elektrodenabschnitte auf der endokardialen Leitung dient primär der Verwirklichung eines Baukastenprinzips für kleinere oder größere Herzen.

In der EP 0 522 693 A1 ist eine zwei separate endokardiale Elektrodenleitungen sowie eine subkutane Flächenelektrode umfassende Anordnung speziell zur atrialen Defibrillation im mehreren Beschaltungsvarianten beschrieben.

In WO 94/03233 wird sowohl für die ventrikuläre als auch die atriale Defibrillation eine Elektrodenanordnung vorgeschlagen, die auf einer im rechten Ventrikel zu verlegenden ersten Leitung eine langgestreckte, spulenförmige Defibrillationselektrode und zwei auf einer zweiten, im Atrium zu verlegenden Leitung angeordnete Abfühl- und Schrittmacherelektroden sowie das Defibrillatorgehäuse als zweite Defibrillationselektrode aufweist.

Aus der WO 95/08366 geht eine Defibrillationselektrodenanordnung hervor, die eine auf eine intrakardial zu positionierende Elektrodenleitung angeordnete Defibrillationselektrode sowie eine extrakardiale Defibrillationselektrode aufweist.

Aus der US 5,235,978 ist eine Defibrillationselektrodenanordnung bekannt, bei der ebenfalls eine im Ventrikel angeordnete intrakardiale Defibrillationselektrode mit einer extrakardialen Patch-Elektrode und einer in der Vena cava inferior angeordneten Elektrode zusammenwirkt.

Die bekannten Defibrillationselektrodenanordnungen sind entweder - sofern sie einen einfachen Aufbau haben und leicht implantierbar sind - hinsichtlich der realisierbaren Funktionen noch relativ beschränkt, oder sie sind -soweit sie komplexere Funktionen erfüllen sollen - kompliziert aufgebaut und aufwendig in der Implantation.

Der Erfindung liegt daher die Aufgabe zugrunde, eine Defibrillationselektrodenanordnung der eingangs genannten Gattung anzugeben, die bei einfachem Aufbau und leichter Implantierbarkeit für die Realisierung einer großen Funktionsvielfalt geeignet ist.

Diese Aufgabe wird durch eine Anordnung mit den Merkmalen des Anspruchs 1 gelöst.

Die Erfindung schließt den Gedanken ein, eine weitgehend universelle Elektrodenanordnung zur Realisierung einer ventrikulären wie auch einer atrialen Defibrillation (bzw. Zweikammer-Defibrillation) sowie der zugehörigen Abfühlfunktion eines automatischen Defibrillators und weiterhin zur Realisierung von Abfühl- und Stimulationsfunktionen eines mit dem Defibrillator kombinierten Schrittmachers mit einer einzigen endokardialen Elektrodenleitung anzugeben. Eine solche Anordnung wird unter Nutzung des Prinzips des zwischen einer nicht wandständigen (flottierenden) ventrikulären Defibrillationselektrode und einer in Anpassung an deren spezifische Gestalt und räumliche Lage positionierten Gegenelektrode ausgebildeten fokussierten Reizimpulsfeldes möglich. Dieses erlaubt die gezielte Vorgabe eines geeigneten Defibrillationsbereiches im reizbaren Herzgewebe auch dann, wenn die Lage der Elektrodenleitung abschnittsweise (speziell im Atrium) zur Realisierung von Zusatzfunktionen über (eine) zusätzliche Elektrode(n) festgelegt ist.

Die zweite Defibrillationselektrode - neben der auf der Elektrodenleitung im rechten Ventrikel angeordneten ersten Defibrillationselektrode - ist in einer bevorzugten Variante durch eine extrakardial angeordnete Flächenelektrode, insbesondere mindestens einen Gehäuseabschnitt des (zu diesem Zweck geeignet zu positionierenden) Defibrillators, gebildet. Die Nutzung des Gerätegehäuses als Gegenelektrode hat den besonderen Vorteil, daß die Implantation einer zusätzlichen Elektrode entfällt.

Bei einer zweiten Variante ist die zweite Defibrillationselektrode durch eine zur Anordnung in der Vena cava superior ausgebildete, insbesondere ebenfalls auf der einzelnen Elektrodenleitung angeordnete, spulenförmige Elektrode gebildet. Die größere Nähe zur ersten Elektrode und die Unabhängigkeit von der Implantationslage des Defibrillators ermöglichen hierbei höhere Reizfeldstärken und eine besonders leichte Verschiebung der Defibrillation-Erregungszone in den atrialen Bereich des Herzgewebes und damit atriale Defibrillation. Auch eine Anordnung der Spulen-Elektrode in der Vena cava inferior oder im Sinus coronarius ist zur Erreichung spezieller Geometrien des Reizfeldes und dadurch vorgegebener Lokalisationen der Defibrillationszone in der Herzwandung möglich.

Beide Varianten können auch in der Weise kombiniert sein, daß - zusätzlich zur Gefäßelektrode als zweite Elektrode-als dritte Defibrillationselektrode eine extrakardial angeordnete Flächenelektrode vorgesehen ist, die wiederum insbesondere durch das Gehäuses des Defibrillators gebildet ist. Die erste und zweite Defibrillationselektrode sind mit Ausgängen des Defibrillators verbunden und haben im Betriebszustand unterschiedliche Polarität in Bezug auf die dritte Elektrode, und die drei Defibrillationselektroden sind räumlich derart zueinander angeordnet, dass sich zwischen ihnen ein räumlich verzerrtes Dipolfeld mit erhöhtem Potentialgradienten oberhalb der Defibrillationsschwelle in einem Bereich der Herzwand bei unterhalb des Defibrillationschwelle liegender Defibrillator-Ausgangsspannung ausbildet. Besonders vorteilhaft ist eine Anordnung, bei der die sich ausbildenden Äquipotentiallinien eine maximale Krümmung im Bereich der Herzwand aufweisen.

Zur genauen Lageeinstellung weist die Elektrodenleitung Abstandshaltermittel auf, die sie nach Einführung in den Ventrikel an der Herzwand abstützen. Zur Gewährleistung eines leichten Einführens der Leitung müssen diese - in an sich bekannter Ausbildung - nach Beendigung der Implantation vom Leitungskörper abspreizbar sein. Zweckmäßigerweise sind sie auch derart extrakorporal einstellbar, dass die Lage der ersten Defibrillationselektrode relativ zur Herzwand nach dem Einführen der Elektrodenleitung veränderbar ist.

In dem Bereich der Elektrodenleitung, der nach Einführung in das Herz im Ventrikel zu liegen kommt, kann in einer für kombinierte Schrittmacher-/Defibrillator-Anordnungen besonders geeigneten Ausführung neben der ersten Defibrillationselektrode im distalen Endabschnitt der Elektrodenleitung eine weitere Elektrode vorgesehen sein. Diese letztere kann als erste und die erste Defibrillationselektrode als zweite ventrikuläre Abfühl- und/oder Schrittmacherelektrode mit dem Defibrillator bzw. einem kombinierten Schrittmacher/Defibrillator verbunden sein. Ist die Nutzung der distalen Elektrode als Abfühlelektrode vorgesehen, so ist deren Ausbildung als Spitzenelektrode, insbesondere mit Mitteln zur Fixierung in der Herzwand, bevorzugt.

Proximal von der ersten Defibrillationselektrode können in einer sowohl für einen automatischen Defibrillator als auch für eine Kombination aus Defibrillator und Zweikammerschrittmacher zweckmäßigen Anordnung auf der Elektrodenleitung zwei weitere Elektroden derart angeordnet sein, daß diese nach Einführung im Atrium, insbesondere in dessen oberem Bereich, liegen.

Mindestens eine dieser atrialen Elektroden - bevorzugt die proximale - ist dann als eine erste Abfühl- und/oder Schrittmachelektrode mit dem Defibrillator bzw. einem kombinierten Schrittmacher/Defibrillator verbunden. In einer bipolaren Ausführung des Sensing- bzw. Stimulationsabschnitts der Elektrodenanordnung sind beide atriale Elektroden als erste und zweite Abfühl- und/oder Schrittmacherelektrode in bipolarer Schaltung mit dem Defibrillator bzw. kombinierten Schrittmacher/Defibrillator verbunden.

In einer bevorzugten unipolaren Anordnung dient speziell das Gerätegehäuse als Abfühl-Referenzelektrode des Defibrillators oder als indifferente Abfühl- und Stimulationselektrode des Schrittmacherteils. Alternativ hierzu kann die zur Anordnung in dem herznahen Gefäß ausgebildete spulenförmige Elektrode als Abfühl- und/oder Schrittmacher-Referenzelektrode mit dem Defibrillator bzw. kombinierten Schrittmacher/Defibrillator verbunden sein.

In einer Ausführung mit besonders großen Variationsmöglichkeiten hinsichtlich der Gestaltung des Reizfeldes weist die erste Defibrillationselektrode eine Mehrzahl von gegeneinander isolierten und einzeln mit dem Defibrillator verbindbaren Teilelektroden auf. Diese können zum einen in zweckmäßiger Weise als Kreisringsegmente einer insgesamt ringförmigen Defibrillationselektrode ausgebildet sein, zum anderen auch als in Längsrichtung der Elektrodenleitung gereihte einzelne, gegeneinander isolierte und ggfs. beabstandete Ringe. In diese Ausführungen können gezielt einzelne Teilelektroden oder auch eine Gruppe aus diesen mit dem Ausgang des Defibrillators verbunden werden, um eine gewünschte Lage und Erstreckung der Defibrillationszone in der Herzwandung zu erreichen.

Weiterhin kann mindestens eine der Defibrillationselektroden, speziell die im Ventrikel angeordnete erste, eine poröse Oberfläche mit fraktaler Oberflächenstruktur aufweisen, die die aktive Elektrodenfläche um mindestens zwei Größenordnungen gegenüber dem sich aus der geometrischen Grundform der Elektrode ergebenden Wert vergrößert, womit eine erhebliche Impedanzverringerung einhergeht.

Der für eine zuverlässige Funktion der atrialen Elektrode(n) als Abfühlelektrode(n) erforderliche Herzwandkontakt wird in vorteilhafter Weise dadurch hergestellt, daß die Elektrodenleitung einen im Betriebszustand im Atrium liegenden V-artig gekrümmten Abschnitt aufweist, der aus zwei Schenkeln gebildet ist, die unter einem spitzen Winkel zusammentreffen und mit den angrenzenden Abschnitten der Elektrodenleitung jeweils einen stumpfen Winkel einschließen. Mindestens eine Abfühl- und/oder Schrittmacherelektrode ist zweckmäßigerweise im wesentlichen am Punkt des Zusammentreffens der Schenkel angeordnet; es können aber auch - falls die Elektrodenleitung zwei atriale Elektroden aufweist - beide Elektroden nahe diesem Punkt angeordnet sein. Diese vorteilhafte Form der Elektrodenleitung wird durch Mittel zur Erzeugung einer Vorspannung (etwa eine entsprechend vorgebogene Feder oder einen Abschnitt aus einer Memeory-Legierung) erzielt, deren Wirkung durch Einführung eines Führungsdrahtes aufgehoben wird, wodurch die Elektrodenleitung zum Einführen gestreckt wird, oder bei Erwärmung der Leitung im Körper selbsttätig eintritt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 schematisch eine Defibrillationselektrodenanordnung nach einer Ausführungsform der Erfindung im implantierten Zustand,
Figur 2 schematisch die Elektrodenleitung einer Defibrillationselektrodenanordnung nach einer zweiten Ausführungsform der Erfindung im implantierten Zustand,
Figur 3 schematisch eine Defibrillationselektrodenanordnung nach einer dritten Ausführungsform der Erfindung im implantierten Zustand,
die Figuren 4 bis 6 schematisch jeweils Abschnitte der Elektrodenleitung von Defibrillationselektrodenanordnungen nach weiteren Ausführungsformen der Erfindung,
die Figuren 7a und 7b die Elektrodenleitung gemäß Fig. 1 (a) im Zustand mit eingeführtem Führungsdraht und (b) in der Form im Betriebszustand und
die Figuren 8a bis 8e Prinzipskizzen von Varianten der Verbindung der einzelnen Elektroden von Defibrillationselektrodenanordnungen gemäß Ausführungsformen der Erfindung mit einem implantierten Defibrillator bzw. kombinierten Defibrillator/Herzschrittmacher.

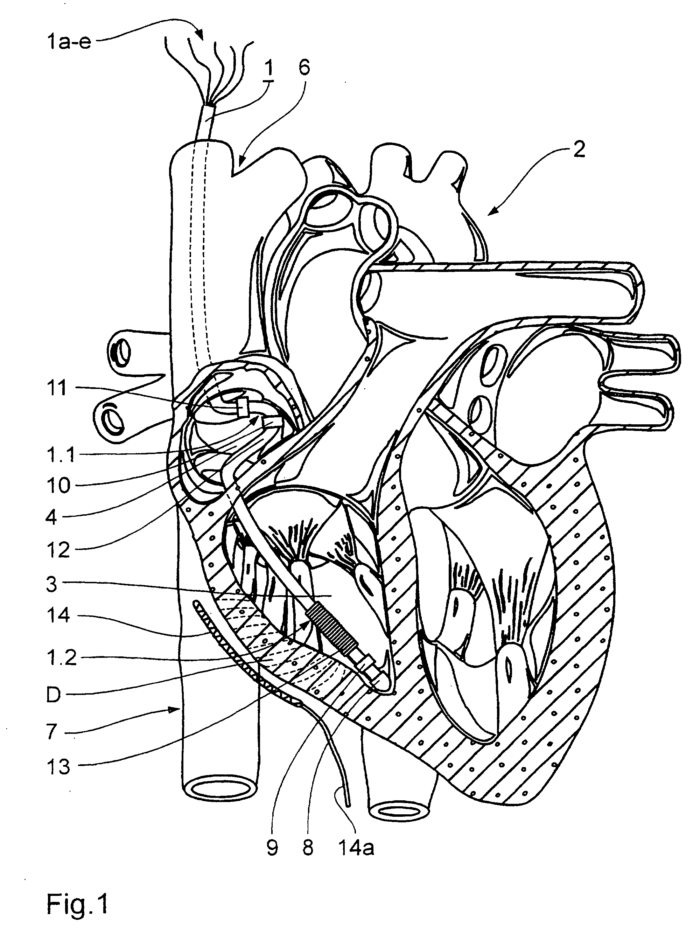

Figur 1 ist eine teilweise geschnittene Darstellung eines Herzens 2 mit einer intrakardial implantierten, mit einem (nicht dargestellten) implantierten Defibrillator oder kombinierten Defibrillator/Herzschrittmacher verbundenen Defibrillationselektrodenanordnung.

Die Elektrodenanordnung weist eine Elektrodenleitung 1 auf, welche durch die obere Hohlvene (Vena cava superior) 6 durch das rechte Atrium 4 bis in den Apexbereich des rechten Ventrikels 3 geführt ist und in einen proximalen (nach der Implantation im Atrium liegenden) Leitungsabschnitt 1.1 und einen distalen (im Ventrikel plazierten) Leitungsabschnitt 1.2 untergliedert ist. Der distale Leitungsabschnitt 1.2 weist an seinem freien Ende zwei eine Spitzenelektrode 8 und in kurzem Abstand proximal von dieser eine Ringelektrode 9 auf. Diese Elektroden 8,9 sind in Abhängigkeit vom Einsatzzweck und der Art des mit der Leitung verbundenen Herzrhythmuskorrekturgerätes in an sich bekannter Weise insbesondere zur Detektion von Herzaktionspotentialen und/oder für eine ventrikuläre Schrittmacher-Stimulierung vorgesehen.

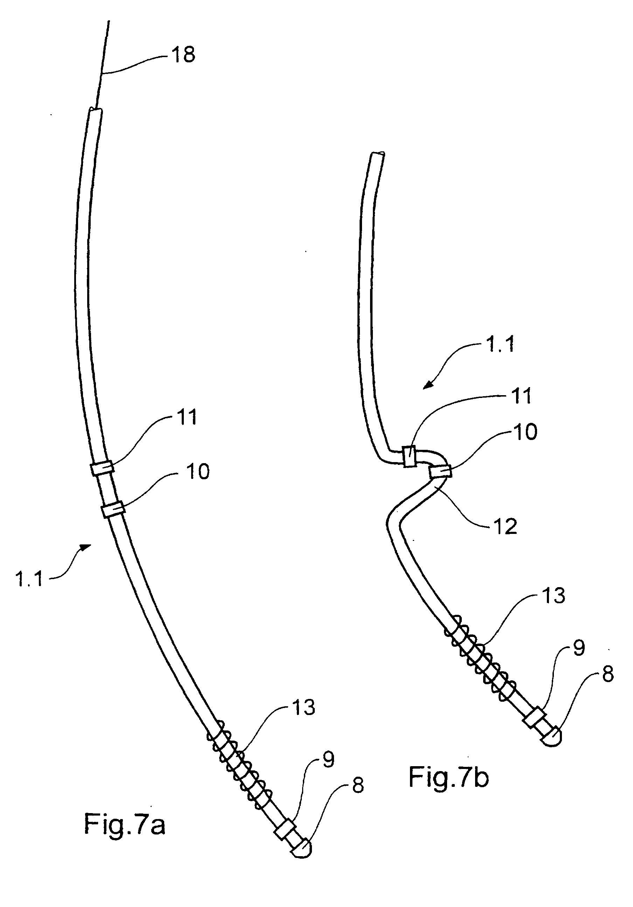

Der mittlere Leitungsabschnitt 1.1 ist im dargestellten implantierten Zustand in Gestalt eines "V" bogenförmig ausgebildet. Diese Form wird durch eine mechanische Vorspannung erreicht, welche der Elektrodenleitung 1 durch (nicht gezeigte) Mittel, wie die Einbindung eines Memory-Metalls oder einer vorgebogenen Feder, abschnittsbezogen aufgeprägt ist. Durch einen während der Implantation im Inneren der Leitung 1 verlaufenden Führungsdraht kann der mittlere Abschnitt 1.1 der Leitung 1 in eine im wesentlichen gestreckte Form überführt werden, was für die Implantation erforderlich ist (vgl. dazu Fig. 7a und 7b). Der V-förmige Abschnitt ist von der Wandung der oberen Hohlvene 6 zur linken Wandung des rechten Atriums 4 gerichtet und kontaktiert dort den Myocard. Im mittleren Leitungsabschnitt 1.1 sind zwei Ringelektroden 10, 11 vorgesehen, die ebenfalls zur Erfassung von Herzaktionspotentialen und/oder zur atrialen Stimulation dienen. Die Elektrode 10 ist im Zenit 12 des Bogens angeordnet, die Elektrode 11 befindet sich proximal hiervon.

Im distalen Abschnitt 1.1 der Elektrodenleitung ist-proximal von den Elektroden 8, 9 - eine langgestreckte spulenförmige Defibrillationselektrode 13 angebracht. Dieser gegenüber ist außerhalb des Herzens, speziell im epikardialen Raum nahe der unteren Hohlvene (Vena cava inferior) 7, eine in an sich bekannter Weise als netz- bzw. gitterförmige Flächenelektrode ausgeführte Defibrillations-Gegenelektrode 14 mit separater Zuleitung 14a vorgesehen. Die Spulen-Elektrode 13 befindet sich in einem ohne Herzwandberührung im Ventrikel liegenden (flottierenden) Bereich der Leitung 1. Bei Ausgabe eines Defibrillationsimpulses auf die Leitung 1 bildet sich zwischen den Elektroden 13, 14 ein elektrisches Schockimpulsfeld aus. Aufgrund dessen bildet sich in dem von ihm mit hinreichend hoher Feldstärke (oberhalb der Defibrillationsschwelle) durchsetzten Bereich D der Wandung des Ventrikels 3 eine (je nach Erstreckung der Gegenelektrode 14) streifen- bis ringförmige Defibrillationszone. Deren Ausmaß und Gestalt ist-neben der Ausgangsspannung des Defibrillators -über die Elektrodenkonfiguration steuerbar.

Am proximalen Ende des in der Figur gezeigten Abschnitts der Elektrodenleitung 1 ist schematisch dargestellt, daß diese fünf einzelne Elektrodenzuleitungen 1a bis 1e aufweist, über die die Elektroden 8 bis 11 und 13 mit dem Defibrillator bzw. Defibrillator/Kardioverter verbunden werden können. Selbstverständlich können - wie auch aus Erläuterungen weiter unten deutlich wird - , je nach konkretem Betriebszustand der Anordnung, einige der Zuleitungen 1a bis 1e aktuell unangeschlossen sein.

Die einzelnen Elektroden können in an sich bekannter Weise und aus bekannten Materialien gefertigt sein. Eine durch eine spezielle Verfahrensführung bei einem Gasphasen-Beschichtungsverfahren (Sputtern) erreichte poröse Oberfläche mit fraktaler Oberflächenstruktur, die die aktive Elektrodenfläche um mindestens zwei Größenordnungen gegenüber der sich aus der geometrischen Grundform der Elektrode ergebenden Oberfläche vergrößert, führt hierbei zu einer vorteilhaften deutlichen Erhöhung der Grenzschichtkapazität gegenüber dem benachbarten Körpergewebe bzw. der umgebenden Körperflüssigkeit und damit zu einem verringerten effektiven Elektrodenwiderstand. Als Oberflächenmaterial kann hierbei vorteilhaft Iridium - etwa auf einen Titan-Träger aufgesputtert Verwendung finden.

Hinsichtlich der - für die Funktion der Anordnung gemäß der Erfindung primären Funktion der zuverlässig wirkungsvollen, energieeffizienten und Gewebsschäden ausschließenden Übertragung von Defibrillationsenergie an das reizbare Herzgewebe ist eine geeignete, ggfs. auf den Patienten zugeschnittene Formgebung, Bemessung und Anordnung der Elektroden 13 und 16 relativ zueinander zur Erreichung einer günstigen Gestalt des Defibrillationsimpulsfeldes von ausschlaggebender Bedeutung. Die Spulenelektrode 13 ist dabei insoweit vorteilhaft, als sie leicht einen gekrümmten Verlauf annehmen kann und dadurch eine leichte "Fokussierung" des Feldes auf bestimmte Bereiche des Myocards ermöglicht. In einer speziellen Ausführung kann sie auch längenveränderlich sein, womit Feldstärke und -gestalt - im Zusammenwirken mit der Bemessung und Plazierung der Flächenelektrode 16 - zusätzlich steuerbar sind.

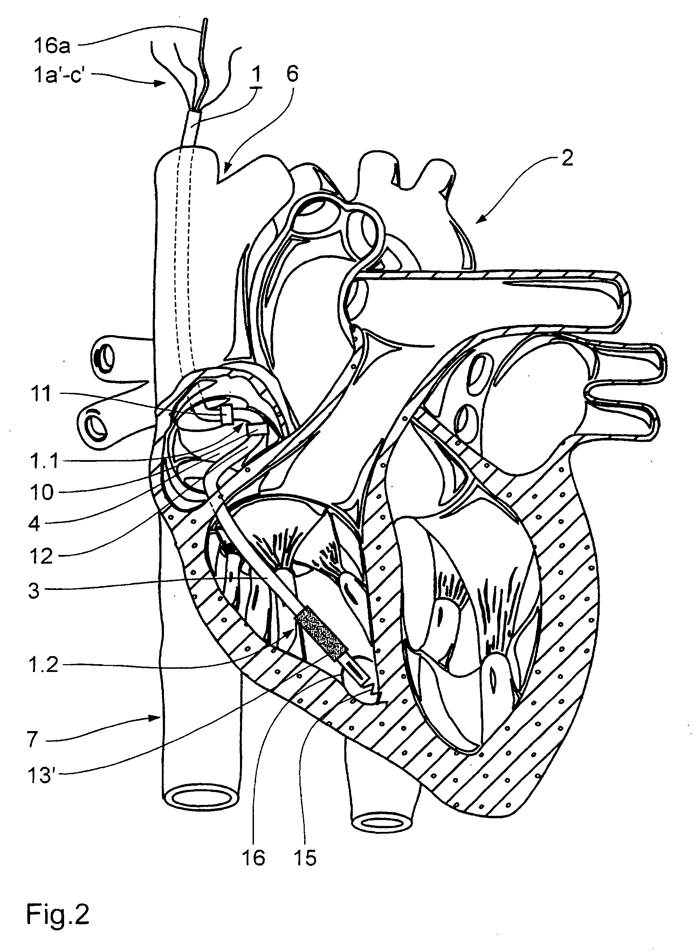

Figur 2 zeigt schematisch eine gegenüber Fig. 1 modifizierte Elektrodenleitung 1'. Die mit Fig. 1 übereinstimmenden Teile sind mit denselben Bezugsziffern wie dort bezeichnet und werden nachfolgend nicht nochmals erläutert.

Bei der vorliegenden Ausführungsform sind die distalen, zur Anordnung im Ventrikel vorgesehenen Abfühl- bzw. Stimulationselektroden entfallen, so daß die Elektrodenleitung auch nur drei Zuleitungen 1a' bis 1c' für die verbliebenen drei Elektroden umfaßt. Weiterhin ist die intraventrikuläre Defibrillationselektrode 13' hier in Form einer Zylindermantel- bzw. langgestreckten Ringelektrode mit poröser Oberfläche ausgebildet, und es sind eine Einschraubspitze 15 am distalen Leitungsende sowie gesteuert ausspreizbare Abstützfüße 16 unmittelbar distal vor der Defibrillationselektrode 13' vorgesehen. Diese Mittel sorgen zusammen für eine sichere Fixierung der Elektrodenleitung und die Einstellung und annähernde Einhaltung eines vorbestimmten Abstandes der Elektrodenoberfläche zur Herzwand auch bei erheblicher körperlicher Aktivität des Patienten. Zur Betätigung der Abstützfüße im Rahmen der Implantation der Leitung ist ein Betätigungsmechanismus vorgesehen, der in der Figur durch den Zugdraht 16a symbolisiert ist.

Schließlich ist bei der in Fig. 2 gezeigten Anordnung keine separate extrakardiale Flächenelektrode vorgesehen; an deren Stelle tritt eine leitende Fläche des (in der Figur nicht gezeigten) Defibrillatorgehäuses. Auch eine Kombination mit einer zusätzlichen Flächenelektrode zu einer Drei-Elektroden-Anordnung (bipolare Anordnung mit Referenzelektrode) ist jedoch möglich. wodurch sich in besonders variabler Weise die Ausbildung des Defibrillationsfeldes den anatomischen und physiologischen Gegebenheiten bei einem speziellen Patienten anpassen, d.h. das Schockimpulsfeld auf vorbestimmte Bereiche des Herzgewebes "fokussieren" läßt.

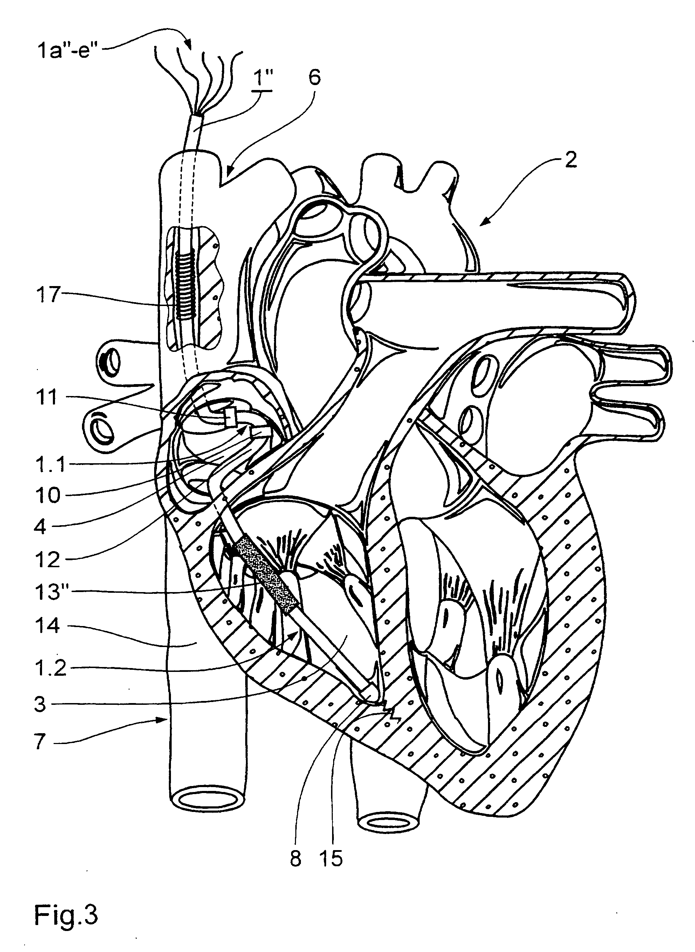

Figur 3 zeigt schematisch eine Defibrillationselektrodenanordnung mit einer weiter abgewandelten Elektrodenleitung 1" nach einer dritten Ausführungsform der Erfindung. Wieder sind die mit Fig. 1 übereinstimmenden Teile sind mit denselben Bezugsziffern wie dort bezeichnet und werden nachfolgend nicht nochmals erläutert.

Bei dieser Leitung 1" ist lediglich eine distale, zur Anordnung im Ventrikel vorgesehene Abfühl- bzw. Stimulationselektrode in Form der Spitzenelektrode 8 vorgesehen, die mit einer Einschraubspitze 15 versehen ist. Die hier wiederum in Form einer Zylindermantel- bzw. langgestreckten Ringelektrode mit poröser Oberfläche ausgeführte intrakardiale Defibrillationselektrode 13" ist in vergleichsweise großem Abstand zum distalen Leitungsende angebracht, d.h. im implantierten Zustand nahe der Herzklappe plaziert. Die Leitung 1'' trägt proximal von den beiden atrialen Elektroden zusätzlich eine nach der Implantation in der Vena cava superior 6 liegende, als Spulenelektrode ausgeführte zweite Defibrillationselektrode 17. Für die somit insgesamt fünf Elektroden sind in der Leitung 1" die fünf Zuleitungen 1a" bis 1e" vorgesehen. Eine zusätzliche extrakardiale Zuleitung wird auch hier - wie bei Fig. 2 -nicht benötigt.

Die räumliche Anordnung der Defibrillationselektroden 13'' und 17 ermöglicht die Erzeugung eines Kardioversionsfeldes speziell im Bereich des atrialen Herzgewebes oder des Sinusknotens, was zur Terminierung bestimmter Arten von Tachykardien (etwa atriale Fibrillation) von Vorteil ist.

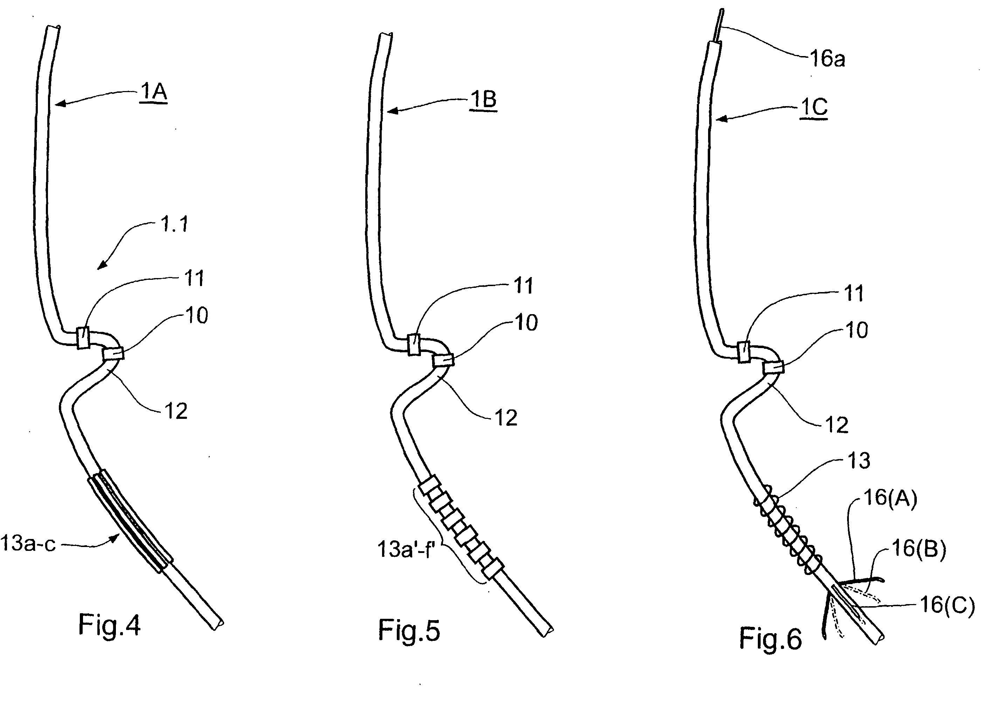

Die Figuren 4 bis 6 zeigen schematisch jeweils Abschnitte von zu weiteren Defibrillationselektrodenanordnungen in Ausführungsformen der Erfindung gehörenden Elektrodenleitungen 1A (Fig. 4), 1B (Fig. 5) und 1C (Fig. 6).

Bei der Leitung 1A nach Fig. 4 sind mehrere zylindersegmentförmig um den Leitungskörper angeordnete separate Defibrillationselektroden vorgesehen, von denen die Teil-Elektroden 13a bis 13c zu sehen sind. Diese separaten Elektroden sind mit getrennten Zuleitungen versehen, so daß sie einzeln oder in Gruppen mit dem Ausgang eines Defibrillators verbunden werden können.

Bei der Leitung 1B nach Fig. 5 sind sechs als Ringelektroden ausgeführte separate Defibrillationselektroden 13a' bis 13f vorgesehen, die ebenfalls mit getrennten Zuleitungen versehen sind, so daß sie einzeln oder in Gruppen mit dem Ausgang eines Defibrillators verbunden werden können. Wie auch mit der Leitung 1(A) nach Fig. 4, kann mit der Leitung (1B) eine differenzierte Lokalisierung und Formung eines Schockimpulsfeldes ereicht werden, ohne daß hierzu - wie in bekannten Anordnungen mit variabler Geometrie des Reizimpulsfeldes - eine Mehrzahl von separat zu verlegenden Elektroden und Zuleitungen nötig wäre.

Die Leitung 1C in Fig. 6 entspricht weitgehend der in Fig. 2 gezeigten Leitung 1' - mit dem wesentlichen Unterschied, daß die Abstützfüße, von denen in der Figur die Füße 16(A) bis 16/C) zu sehen sind, gegenüber dem Leitungskörper winkelverstellbar sind, wodurch sich die Lage der Defibrillationselektrode 13 innerhalb des Ventrikels differenzierter einstellen läßt. Dies ist insbesondere bei einer Ausführung ohne Einschraubspitze vorteilhaft, bei der über eine Winkelverstellung der Abstützfüße auch die Lage der Elektrode in Längsrichtung des Ventrikels beeinflußt werden kann.

Die Erfindung beschränkt sich in ihrer Ausführung nicht auf die vorstehend angegebenen Ausführungsbeispiele. Vielmehr ist eine Vielzahl von weiteren Abwandlungen denkbar, die sich insbesondere durch Verzicht auf einzelne Elemente und/oder andere Kombinationen von in verschiedenen Figuren dargestellten Elementen in einer Anordnung ergeben können.

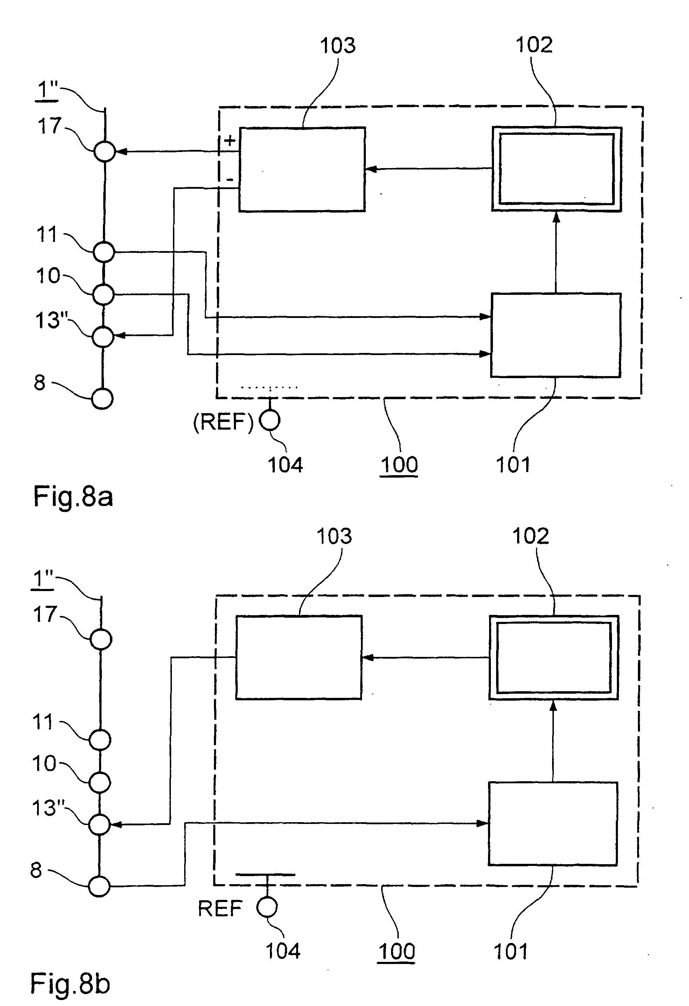

In den Figuren 8a bis 8e sind in Prinzipskizzen Varianten der Verbindung der einzelnen Elektroden einer Elektrodenanordnung gemäß einer Ausführungsform der Erfindung - nämlich der Elektrodenleitung 1" nach Fig. 3 - mit einem implantierten Defibrillator 100 bzw. kombinierten Defibrillator/Herzschrittmacher 100' gezeigt, mit denen an Beispielen die Vielfalt der, jeweils für bestimmte Einsatzbedingungen zweckmäßigen, Schaltungsmöglichkeiten illustriert werden soll.

In Fig. 8a sind die atrialen Elektroden 10 und 11 der Elektrodenleitung 1" als Abfühlelektroden für das Auftreten tachykarder Arrhyhtmien in bipolarer Schaltung mit einer Eingangsstufe 101 eines Defibrillators 100 verbunden, die ausgangssseitig mit einer Steuereinheit 102 verbunden ist. Diese wiederum ist ausgangsseitig mit einer Ausgangsstufe 103 verbunden, die bei Erfassung von Herzsignalen, die eine durch einen Schockimpuls zu terminierende Arrhythmie anzeigen, einen entsprechenden Schockimpuls -ebenfalls bipolar-über die Defibrillationselektroden 13, 17 an das Herzgewebe im Bereich des Atriums anlegt. Eine leitende Gehäusefläche 104 des Defibrillatorgehäuses kann hierbei zusätzlich als Referenzelektrode REF wirken und -je nach Position des Defibrillators relativ zum Herz -spürbar die Feldgestalt beeinflussen. Die ventrikuläre Spitzenelektrode 8 ist hier nicht angeschlossen.

Gemäß Fig. 8b bilden diese Spitzenelektrode 8 und die Gehäuseelektrode 104 (als Referenzelektrode REF) eine unipolare Abfühlanordnung für ventrikuläre Herzsignale und die intraventrikuläre Defibrillationselektrode 13 sowie wiederum die Gehäuseelektrode 104 eine ebenfalls unipolare Schokkelektrodenkonfiguration zum Anlegen eines im Bereich des rechten Ventrikels wirkenden Kardioversionsimpulses. Die übrigen Elektroden werden bei diesem Beispiel nicht benutzt.

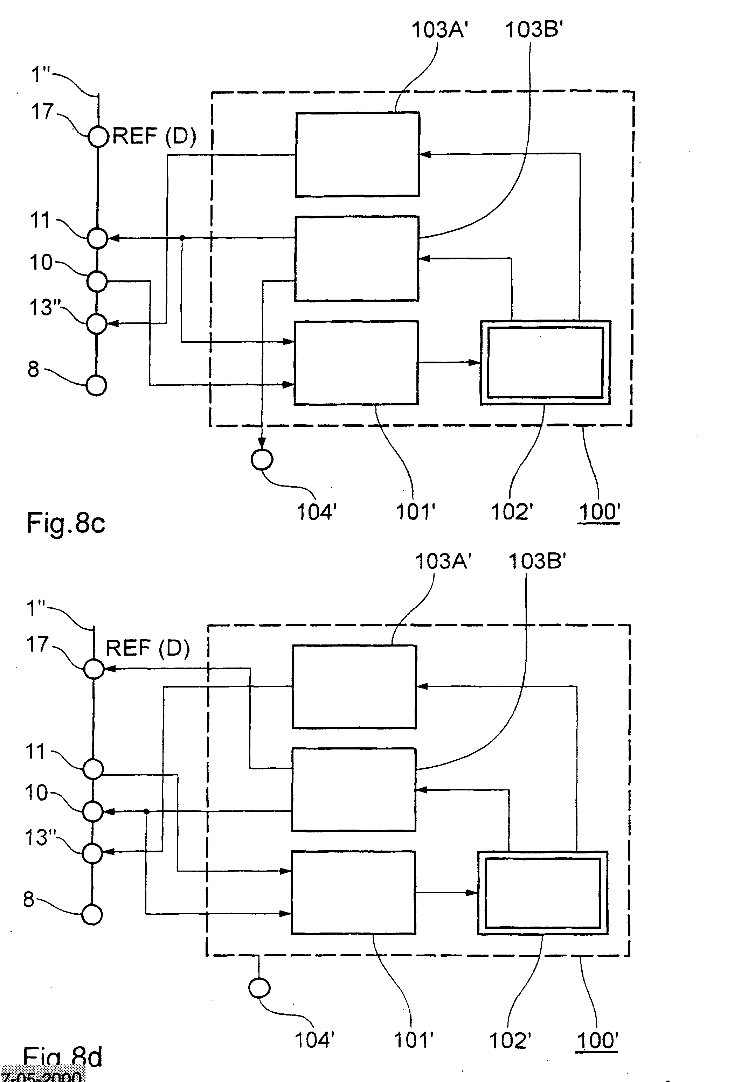

Gemäß Fig. 8c sind die atrialen Elektroden 10 und 11 als universelle Abfühlelektroden zur Erfassung der atrialen Herzaktionspotentiale als Eingangsssignale zur Steuerung eines kombinierten Herzschrittmachers/Defibrillators 100' in bipolarer Schaltung mit dessen Eingangsstufe 101' verbunden, die ausgangsseitig mit einer - natürlich entsprechend dem erweiterten Einsatzzweck des Gerätes ausgebildeten Steuereinheit 102' verbunden ist. Die letztere ist ausgangssseitig mit einer Defibrillator-Ausgangsstufe 103A' und einer Herzschrittmacher-Ausgangsstufe 103B' verbunden, die entsprechend der Auswertung der über die atrialen Elektroden 10, 11 erfaßten Herzsignale bedarfsweise Schrittmacherimpulse oder einen Kardioversionsschock liefern. Die Defibrillator-Ausgangsstufe 103A' ist unipolar mit der ventrikulären Defibrillationselektrode 13 verbunden, während die Vena-cava-Elektrode 17 im Falle einer Kardioversion hier als Referenzelektrode REF(D) wirkt. Eine leitende Gehäusefläche 104' des Kombinationsgerätes 100' sowie die proximale Elektrode 11 der beiden Atriumelektroden sind mit den Ausgängen der Schrittmacher-Endstufe 103B' verbunden, so daß Schrittmacherimpulse über diese ausgegeben werden.

Fig. 8d zeigt eine Abwandlung dieser Schaltung, die sich von der letzteren dadurch unterscheidet, daß die Vena-cava-Spule 17 neben der distalen Atrium-Elektrode 10 als Schrittmacherelektrode bei einer bipolaren Stimulation dient. Statt der distalen kann auch die proximale Atriumelektrode 10 mit dem Schrittmacherausgang verbunden sein.

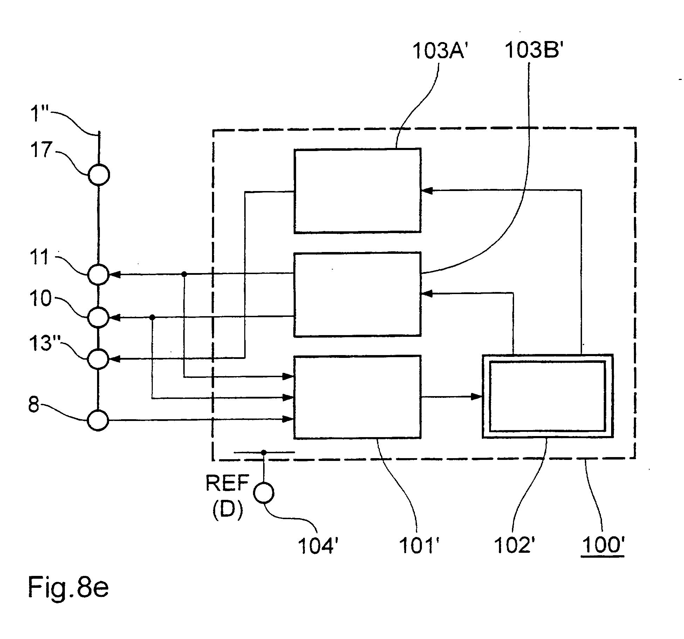

Eine weitere Modifikation zeigt Fig. 8e: Hier erfolgt die atriale Schrittmacher-Stimulation ebenso wie die Erfassung der atrialen Herzaktionen für die Steuerung des Schrittmacherteils über die beiden atrialen Elektroden 10, 11. Hingegen wird ein Defibrillationsschock zur Beendigung einer ventrikulären Fibrillation auf ein über die ventrikuläre Spitzenelektrode 8 aufgenommenes ventrikuläres Tachykardiesignal hin über die ventrikuläre Defibrillationselektrode 13 abgegeben, wobei das Gerätegehäuse 104' als Referenzelektrode REF(D) wirkt.

Bei zusätzlichem Einsatz einer subkutanen Flächenelektrode (wie in Fig. 1 gezeigt) kann diese neben dem Einsatz als echte dritte Elektrode mit unabhängigem Potential auch elektrisch parallel zur Vena-cava-Spule 17 oder der Geräteelektrode 104 bzw. 104' geschaltet sein.

Entsprechende und weitere, für den Fachmann anhand der Grundgedanken der Erfindung ableitbare Schaltungsvarianten sind für die übrigen oben beschriebenen und weitere im Schutzbereich der Ansprüche liegende Elektrodenkonfigurationen möglich. Insbesondere sind ohne weiteres Varianten realisierbar, mit denen sowohl ein Abfühlen von Herzaktionen als Eingangssignale für einen Zweuikammerschrittmacher als auch die Ausgabe von dessen Stimulationsimpulsen neben der Übertragung von Defibrillationsmpulsen - über Elektroden auf einer einzigen Elektrodenleitung möglich wird, wodurch das oben erläuterte Konzept des Ein-Leitungs-Defibrillators in vorteilhafter Weise mit dem des (als solchen bekannten) Ein-Leitungs-Zweikammerschrittmachers verknüpft wird. Hierfür eignet sich besonders die Anordnung nach Fig. 1, die neben der ventrikulären Defibrillationselektrode zwei ventrikuläre Abfühl- und/oder Schrittmacherlektroden aufweist.

## Patentansprüche

1. Defibrillationselektrodenanordnung zur Verbindung mit einem implantierbaren Defibrillator (100; 100'), mit mindestens einer ersten, auf einer intrakardial zu positionierenden Elektrodenleitung (1; 1'; 1"; 1A; 1B; 1C) angeordneten, Defibrillationselektrode (13; 13'; 13"; 13a-c; 13a'-f') sowie einer zweiten Defibrillationselektrode (14; 17), wobei die erste Defibrillationselektrode als zylinder- oder zylindersegmentförmige Elektrode ausgebildet und in derart vorbestimmtem Abstand vom distalen Ende der Elektrodenleitung angeordnet ist, dass sie nach Einführung der Elektrodenleitung ohne Herzwandberührung frei in einem Herzen (2), insbesondere im Ventrikel (3), liegt und die zweite Defibrillationselektrode (14; 17) außerhalb des Ventrikels angeordnet ist, derart, dass sich im Betriebszustand des Defibrillators in der Herzwand eine streifen- bis ringförmige Defibrillationszone (D) ausbildet, und auf der Elektrodenleitung weiterhin mindestens eine Abfühl- und/oder Schrittmacherelektrode (10, 11) derart angeordnet ist, dass sie nach Einführung der Elektrodenleitung im Atrium (3) des Herzens liegt, **dadurch gekennzeichnet, dass** die Elektrodenleitung (1C) nichtleitende Abstandshaltermittel (16; 16[A], 16[B], 16[C]) aufweist, die die Elektrodenleitung nach Einführung in den Ventrikel an der Herzwand abstützen und dass die Abstandshaltermittel (16[A], 16[B], 16[C]) extrakorporal einstellbar sind, derart, dass die Lage der ersten Defibrillationselektrode (13) relativ zur Herzwand nach dem Einführen der Elektrodenleitung (1 C) veränderbar ist.

2. Defibrillationselektrodenanordnung nach Anspruch 1, **dadurch gekennzeichnet, dass** die zweite Defibrillationselektrode durch eine extrakardial angeordnete Flächenelektrode (14) oder mindestens einen Abschnitt (104; 104') des Gehäuses des implantierbaren Defibrillators (100; 100') gebildet ist.

3. Defibrillationselektrodenanordnung nach Anspruch 2, **dadurch gekennzeichnet, dass** die zweite Defibrillationselektrode durch eine zur Anordnung in einem herznahen Blutgefäß (7) ausgebildete, insbesondere auf der Elektrodenleitung (1") angeordnete und spulenförmig ausgeführte, Elektrode (17) gebildet ist.

4. Defibrillationselektrodenanordnung nach Anspruch 3, **dadurch gekennzeichnet, dass** als dritte Defibrillationselektrode eine extrakardial angeordnete Flächenelektrode (14; 104; 104') vorgesehen ist, die insbesondere durch einen Abschnitt des Gehäuses des implantierbaren Defibrillators (100; 100') gebildet ist, wobei die erste und zweite Defibrillationselektrode (13, 17) mit Ausgängen des Defibrillators verbunden sind und in dessen Betriebszustand unterschiedliche Polarität in Bezug auf die dritte Elektrode aufweisen und die erste bis dritte Defibrillationselektrode räumlich derart zueinander angeordnet sind, dass sich zwischen ihnen ein räumlich verzerrtes Dipolfeld mit erhöhtem Potentialgradienten oberhalb der Defibrillationsschwelle in einem Bereich der Herzwand bei unterhalb der Defibrillationsschwelle liegender Defibrillator-Ausgangsspannung ausbildet.

5. Defibrillationselektrodenanordnung nach Anspruch 4, **dadurch gekennzeichnet, dass** die erste bis dritte Defibrillationselektrode derart räumlich angeordnet sind, dass die sich ausbildenden Äquipotentiallinien eine maximale Krümmung im Bereich der Herzwand aufweisen.

6. Defibrillationselektrodenanordnung nach einem der vorangehenden Ansprüche, **dadurch gekennzeichnet, dass** im distalen Endbereich der Elektrodenleitung (1; 1) mindestens eine weitere Elektrode (8, 9) vorgesehen ist, wobei diese Elektrode (8, 9) als eine erste und die erste Defibrillationselektrode (13) als zweite ventrikuläre Abfühl- und/oder Schrittmacherelektrode mit dem Defibrillator (100) bzw. einem kombinierten Schrittmacher/Defibrillator (100') verbunden sind.

7. Defibrillationselektrodenanordnung nach einem der vorangehenden Ansprüche, **dadurch gekennzeichnet, dass** proximal von der ersten Defibrillationselektrode (13; 13'; 13") auf der Elektrodenleitung in einem derartigen Abstand zu dieser zwei weitere Elektroden (10, 11) angeordnet sind, dass diese nach Einführung der Elektrodenleitung im Atrium (3), insbesondere in dessen oberem Bereich, liegen, wobei mindestens eine der atrialen Elektroden (10,11) als eine erste Abfühl- und/oder Schrittmacherelektrode mit dem Defibrillator (100) oder einem kombinierten Schrittmacher/Defibrillator (100') verbunden ist.

8. Defibrillationselektrodenanordnung nach einem der Ansprüche 3 bis 9, **dadurch gekennzeichnet, dass** die zur Anordnung im herznahen Gefäß (7) ausgebildete spulenförmige Elektrode (17) als Abfühl- und/oder Schrittmacher-Referenzelektrode mit dem Defibrillator (100) oder kombinierten Schrittmacher/Defibrillator (100') verbunden ist.

9. Defibrillationselektrodenanordnung nach einem der vorangehenden Ansprüche, **dadurch gekennzeichnet, dass** die erste Defibrillationselektrode eine Mehrzahl von gegeneinander isolierten und einzeln mit dem Defibrillator verbindbaren Teil-Elektroden (13a-13c; 13a'-13f') aufweist, die als Zylindermantelsegmente (13a-13c) oder als in Längsrichtung der Elektrodenleitung (1B) gereihte Ringe (13a'-13f') ausgebildet sind.

10. Defibrillationselektrodenanordnung nach einem der vorangehenden Ansprüche, **dadurch gekennzeichnet, dass** die Elektrodenleitung (1; 1'; 1") einen im Betriebszustand im Atrium liegenden gekrümmten Abschnitt (1.1) hat, der zwei Schenkel aufweist, welche unter einem spitzen Winkel in einem Punkt (12) zusammentreffen und mit den angrenzenden Abschnitten der Elektrodenleitung jeweils einen Winkel ≥ 90° einschließen, wobei die Abfühl- und/oder Schrittmacherelektrode (10) im wesentlichen am Punkt (12) des Zusammentreffens der Schenkel angeordnet ist.

## Claims

1. A defibrillation electrode arrangement for connection to an implantable defibrillator (100; 100') comprising at least a first defibrillation electrode (13; 13'; 13"' 13a-c; 13a'-f') arranged on an electrode line (1; 1'; 1"; 1A; 1B; 1C) to be positioned intracardially, and a second defibrillation electrode (14; 17), wherein the first defibrillation electrode is in the form of an electrode in the shape of a cylinder or a segment of a cylinder and is arranged at a predetermined spacing from the distal end of the electrode line such that after introduction of the electrode line it is disposed without touching the wall of the heart free in a heart (2), in particular in the ventricle (3), and the second defibrillation electrode (14; 17) is arranged outside the ventricle in such a way that in the operating condition of the defibrillator formed in the wall of the heart is a strip-shaped to ring-shaped defibrillation zone (D) and in addition at least one sensing and/or pacemaker electrode (10, 11) is arranged on the electrode line in such a way that after introduction of the electrode line it is disposed in the atrium (3) of the heart, **characterised in that** the electrode line (1C) has nonconducting spacer means (16; 16[A], 16[B], 16[C]) which support the electrode line after introduction into the ventricle against the wall of the heart and that the spacer means (16[A], 16[B], 16[C]) can be extracorporeally adjusted in such a way that the position of the first defibrillation electrode (13) relative to the wall of the heart can be varied after introduction of the electrode line (1C).

2. A defibrillation electrode arrangement according to claim 1 **characterised in that** the second defibrillation electrode is formed by an extracardially arranged surface electrode (14) or at least a portion (104; 104') of the housing of the implantable defibrillator (100; 100').

3. A defibrillation electrode arrangement according to claim 2 **characterised in that** the second defibrillation electrode is formed by an electrode (17) which is designed for arrangement in a blood vessel (7) near the heart and which in particular is arranged on the electrode line (1') and which is of a coil-shaped configuration.

4. A defibrillation electrode arrangement according to claim 3 **characterised in that** the third defibrillation electrode is an extracardially arranged surface electrode (14; 104; 104') which is formed in particular by a portion of the housing of the implantable defibrillator (100; 100'), wherein the first and second defibrillation electrodes (13, 17) are connected to outputs of the defibrillator and in the operating condition thereof have different polarities in relation to the third electrode and the first to third defibrillation electrodes are arranged spatially relative to each other in such a way that formed between them is a spatially distorted dipole field with an increased potential gradient above the defibrillation threshold in a region of the wall of the heart at a defibrillator output voltage which is below the defibrillation threshold.

5. A defibrillation electrode arrangement according to claim 4 **characterised in that** the first to third defibrillation electrodes are arranged spatially such that the equipotentials formed have a maximum curvature in the region of the wall of the heart.

6. A defibrillation electrode arrangement according to one of the preceding claims **characterised in that** at least one further electrode (8, 9) is provided in the distal end region of the electrode line (1; 1"), wherein said electrode (8, 9) as a first ventricular sensing and/or pacemaker electrode and the first defibrillation electrode (13) as a second ventricular sensing and/or pacemaker electrode are connected to the defibrillator (100) or a combined pacemaker/defibrillator (100') respectively.

7. A defibrillation electrode arrangement according to one of the preceding claims **characterised in that** arranged proximally of the first defibrillation electrode (13; 13'; 13") on the electrode line at a spacing relative thereto are two further electrodes (10, 11), which spacing is such that after introduction of the electrode line they are disposed in the atrium (3), in particular in the upper region thereof, wherein at least one of the atrial electrodes (10, 11) is connected as a first sensing and/or pacemaker electrode to the defibrillator (100) or a combined pacemaker/defibrillator (100').

8. A defibrillation electrode arrangement according to one of claims 3 to 9 **characterised in that** the coil-shaped electrode (17) designed to be arranged in the vessel (7) near the heart is connected as a sensing and/or pacemaker reference electrode to the defibrillator (100) or combined pacemaker/defibrillator (100').

9. A defibrillation electrode arrangement according to one of the preceding claims **characterised in that** the first defibrillation electrode has a plurality of mutually insulated electrode portions (13a-13c; 13a'-13f') which can be individually connected to the defibrillator and which are in the form of segments of a cylindrical surface (13a-c) or in the form of rings (13a'-13f) arranged in a row in the longitudinal direction of the electrode line (1B).

10. A defibrillation electrode arrangement according to one of the preceding claims **characterised in that** the electrode line (1; 1'; 1") has a curved portion (1.1) which in the operating condition is disposed in the atrium and which has two limbs which meet at an acute angle at a point (12) and each include an angle ≥ 90° with the adjoining portions of the electrode line, wherein the sensing and/or pacemaker electrode (10) is arranged substantially at the point (12) at which the limbs meet.

## Revendications

1. Dispositif à électrodes de défibrillation destiné à être relié à un défibrillateur implantable (100; 100'), comportant au moins une première électrode de défibrillation (13; 13'; 13"; 13a-c; 13a'-f'), disposée dans un conducteur d'électrode (1; 1'; 1"; 1A; 1B; 1C) devant être positionnée d'une manière intracardiaque, ainsi qu'une seconde électrode de défibrillation (14; 17), la première électrode de défibrillation étant agencée sous la forme d'une électrode de forme cylindrique ou en forme de segment cylindrique et étant disposée à une distance prédéterminée telle de l'extrémité distale du conducteur d'électrode qu'après l'introduction du conducteur d'électrode sans contact avec la paroi cardiaque, cette électrode est située librement à l'intérieur d'un coeur (2), notamment dans le ventricule (30) et la seconde électrode de défibrillation (14; 17) est disposée à l'extérieur du ventricule de telle sorte qu'il s'établit, dans l'état de fonctionnement du défibrillateur, une zone de défibrillation en forme de bande allant jusqu'à une forme annulaire (D), dans la paroi du coeur, et au moins une électrode de détection et/ou de stimulation cardiaque (10, 11) est disposée dans le conducteur d'électrode de telle sorte qu'après l'introduction du conducteur d'électrode, cette électrode est située dans l'oreillette (3) du coeur, **caractérisé en ce que** le conducteur d'électrode (1C) comporte des moyens d'entretoisement non conducteurs (16; [16A] , [16B] , [16C]), qui supportent le conducteur d'électrode après l'introduction dans le ventricule, contre la paroi cardiaque et que les moyens d'entretoisement ([16A], [16B], [16C]) peuvent être réglés d'une manière extracorporelle de telle sorte que la position de la première électrode de défibrillation (13) peut être modifiée par rapport à la paroi du coeur après l'introduction du conducteur d'électrode (1C).

2. Dispositif à électrodes de défibrillation selon la revendication 1, **caractérisé en ce que** la seconde électrode de défibrillation est formée par une électrode de surface (14) disposée de façon extracardiaque ou par au moins une partie (104; 104') du boîtier du défibrillateur implantable (100; 100').

3. Dispositif à électrodes de défibrillation selon la revendication 2, **caractérisé en ce que** la seconde électrode de défibrillation est formée par une électrode (17) agencée en forme de bobine, qui est formée pour être disposée dans un vaisseau sanguin (7) proche du coeur et notamment est disposée sur le conducteur d'électrode (1").

4. Dispositif à électrodes de défibrillation selon la revendication 3, **caractérisé en ce qu'**il est prévu comme troisième électrode de défibrillation une électrode de surface (14; 104; 104') disposée de façon extracardiaque et qui est formée notamment par une partie du boîtier du défibrillateur implantable (100; 100'), les première et seconde électrodes de défibrillation (13, 17) étant reliées à des sorties du défibrillateur et, dans l'état de fonctionnement de ce dernier, possédant une polarité différente de la troisième électrode, et les première à troisième électrodes de défibrillation étant disposées spatialement les unes par rapport aux autres de telle sorte qu'il se forme entre elles un champ dipolaire présentant une distorsion spatiale avec un gradient de potentiel accru au-dessus du seuil de défibrillation dans une zone de la paroi cardiaque, lorsque la tension de sortie du défibrillateur est inférieure au seuil de défibrillation.

5. Dispositif à électrodes de défibrillation selon la revendication 4, **caractérisé en ce que** les première à troisièmes électrodes de défibrillation sont disposées spatialement de telle sorte que les lignes équipotentielles, qui se forment, possèdent une courbure maximale dans la zone de la paroi cardiaque.

6. Dispositif à électrodes de défibrillation selon l'une des revendications précédentes, **caractérisé en ce qu'**au moins une autre électrode (8, 9) est prévue dans la zone d'extrémité distale du conducteur d'électrode (1; 1"), cette électrode et la première électrode de défibrillation (13) étant reliées respectivement en tant que première et seconde électrodes de détection et/ou de stimulation ventriculaire, respectivement au défibrillateur (100) ou à un ensemble combiné stimulateur/défibrillateur (100').

7. Dispositif à électrodes de défibrillation selon l'une des revendications précédentes, **caractérisé en ce que** deux autres électrodes (10, 13) sont disposées sur le côté proximal de la première électrode de défibrillation (13; 13'; 13") dans le conducteur d'électrode, à une distance telle de cette électrode qu'après l'introduction du conducteur d'électrode, ces électrodes sont situées dans l'oreillette (3), notamment dans la partie supérieure de cette dernière, auquel cas au moins l'une des électrodes atriales (10, 11) est reliée en tant que première électrode de détection et/ou de stimulation, au défibrillateur (100) ou à un ensemble combiné stimulateur/défibrillateur (100').

8. Dispositif à électrodes de défibrillation selon l'une des revendications 3 à 9, **caractérisé en ce que** l'électrode (17) en forme de bobine, qui est conçue pour être disposée dans le vaisseau (7) proche du coeur, est reliée en tant qu'électrode de référence de détection et/ou de stimulation au défibrillateur (100) ou à l'ensemble combiné stimulateur/défibrillateur (100').

9. Dispositif à électrodes de défibrillation selon l'une des revendications précédentes, **caractérisé en ce que** la première électrode de défibrillation comporte une multiplicité d'électrodes partielles (13a-13c; 13a'-13f') isolées les unes des autres et pouvant être reliées individuellement au défibrillateur et qui sont agencées sous la forme de segments d'enveloppe cylindrique (13a-13c) ou sous la forme d'anneaux (13a'-13f') rangés dans la direction longitudinale du conducteur d'électrode (1B).

10. Dispositif à électrodes de défibrillation selon l'une des revendications précédentes, **caractérisé en ce que** le conducteur d'électrode (1; 1'; 1") possède une section (1.1) qui dans l'état de fonctionnement est située dans l'oreillette et qui possède deux branches qui convergent en faisant un angle aigu en un point (12) et définissent avec les sections contiguës du conducteur d'électrode respectivement un angle ≥ 90°, l'électrode de détection et/ou de stimulation (10) étant disposée essentiellement au niveau du point de réunion des branches.
